# EUROPEAN PATENT APPLICATION

(11) **EP 1 308 141 A1**
(43) Date of publication of application: **07.05.2003**
(21) Application number: 01830677.9
(22) Date of filing: 30.10.2001
(51) Int. Cl.: A61F 2/34

(54) **An acetabular cup for a hip joint prosthesis**

(71) Applicant: Permedica S.p.a., 23807 Merate (Lecco) (IT)
(72) Inventor: Perego, Marco, 23807 Merate (Lecco) (IT)
(74) Representative: Siniscalco, Fabio

(57) **Abstract**

A socket (1) for a hip joint prosthesis is provided with a plurality of meridian incisions (7) which define as many segments (8) elastically flexible in a radial direction. The inner surface (5) of the socket (1) is provided with one or more grooves (14, 18) parallel to the proximal rim, wherein these grooves receive corresponding reliefs (29, 34) of a joint insert (20). The socket additionally comprises one or more frustoconical portions (13, 17) which form bearing surfaces for corresponding frustoconical portions (28, 33) of the joint insert (20). The inner surface 5 of the socket (1) bounds a polygonal seat (16) which is adapted to engage with a polygonal portion (31) of the joint insert (20) by means of a form fit.

## Description

The subject of the present invention is an expansible acetabular cup for a hip joint prosthesis.

Hip joint prostheses are known which comprise an expansible acetabular cup and a cap-shaped insert. Expansible acetabular cups are generally in the shape of a cap with a plurality of incisions which extend along meridians of said cap and sub-divide the body of the socket into a plurality of segments, the flexibility of which renders the socket radially expansible.

The acetabular cup is intended to be inserted in a suitable seat, produced by means of surgical intervention, at the acetabulum. Inside the cup a cavity is formed, in which is inserted the cap-shaped joint insert, which acts as the acetabulum, receiving the substantially spherical head of a femoral prosthesis.

Acetabular cups are generally produced in a biocompatible and osteo-integrable material, for example titanium, and some of its alloys. The cap-shaped insert is customarily produced in a material having a high resistance to wear and a low coefficient of friction in connection with the aforesaid head of the femoral prosthesis. The material commonly used for the production of inserts for acetabular cups is high molecular density polyethylene.

The acetabular cups of the prior art have some problems and drawbacks, in particular with regard to the connection between the bone of the patient's pelvis and the acetabular cup and also the connection between cup and insert. These problems are mainly linked to the specific properties and the diversity of the coupled materials and also to the mechanical stresses to which they are subjected.

With regard to the polyethylene inserts, in the course of time, play may be observed between the insert and the acetabular cup, due to wear and/or deformation of the polyethylene. Extreme consequences of such play may be the rotation of the insert within the cavity of the acetabular cup and also the expulsion of the insert because of the elastic properties of the material.

The play, occurring in the course of time, besides negatively affecting the actual functioning of the prosthesis, also involves an alteration in the play of forces provided between the bony structure and the individual components of the hip joint prosthesis, involving secondary damage of varying nature.

The strength and reliability of the connection between the acetabular cup and the surrounding bony tissue depends very much on the penetration of the roughened or toothed outer surface of the segments into the bony tissue. In the case of a very hardened, for example sclerotic, bony tissue, complete insertion of the acetabular cup into the seat milled into the pelvis is often not possible, so that the toothing, customarily present in the proximal region of the cup, does not reach the natural bone sufficiently.

The connection between pelvic bone, expansible acetabular cup and joint insert has the particular feature that when the cup is widely expanded, its connection with the surrounding bony tissue is very tight, but the connection with the polyethylene insert often exhibits unwanted play. On the other hand, when the cup is less expanded, it often does not engage properly with the bone of the pelvis.

In the systems of the prior art, the joint insert, during insertion into the acetabular cup, may undergo irreversible deformation, and in extreme cases protruding pieces may be sheared off. This fact, in its turn, prejudices the correct functioning of the hip joint prosthesis.

The aforesaid reasons for the damage to the joint inserts and for the occurrence of unwanted play in the connection between bone, acetabular cup and joint insert constitute a very serious problem in so far as further surgical intervention on the patient to restore the correct functioning of the prosthesis becomes inevitable.

The aim of the present invention is therefore that of solving the problems described above, by providing an expansible acetabular cup for a hip joint prosthesis having characteristics such as to guarantee a secure and reliable connection between the socket and the joint insert.

Within the framework of the above-mentioned aim, a particular purpose of the present invention is that of providing a socket equipped with a structure such as to additionally obtain optimum anchorage in the bone of the pelvis.

The above aim is fulfilled by means of a socket for a hip joint prosthesis according to claim 1 and by means of a joint insert according to claim 28.

For greater understanding of the invention, there now follows a description of one of its non-limiting exemplary embodiments illustrated in the appended drawings, in which:
Figure 1 is a lateral/proximal perspective view of the socket according to the invention;
Figure 2 is a distal view of the acetabular cup according to the invention;
Figure 3 is a lateral view of the socket according to the invention;
Figure 4 is a proximal view of the socket according to the invention;
Figure 5 shows a section through the socket of Figure 2 along the line V-V;
Figure 6 is an enlarged illustration of the detail VI in Figure 5;
Figure 7 is a proximal view of the joint insert according to the invention;
Figure 8 is a side view of the joint insert according to the invention;
Figure 9 shows a section through the joint insert of Figure 7 along the line IX-IX;
Figure 10 shows a section through the joint insert' of Figure 8 along the line X-X;
Figure 11 is an enlarged illustration of the detail XI in Figure 9;
Figure 12 shows, in cross-section, the connection between a socket according to the invention and a joint insert according to the invention;
Figure 13 shows diagrammatically a hip joint prosthesis with an acetabular cup according to the invention.

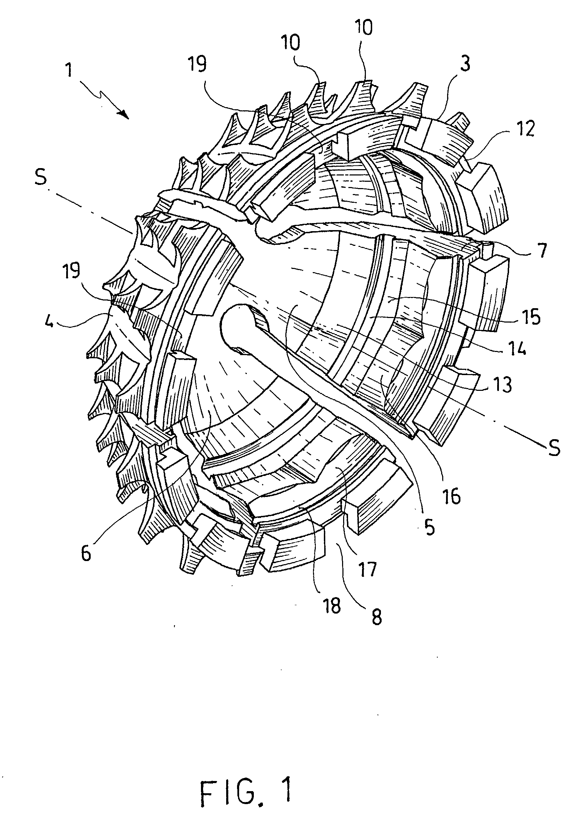

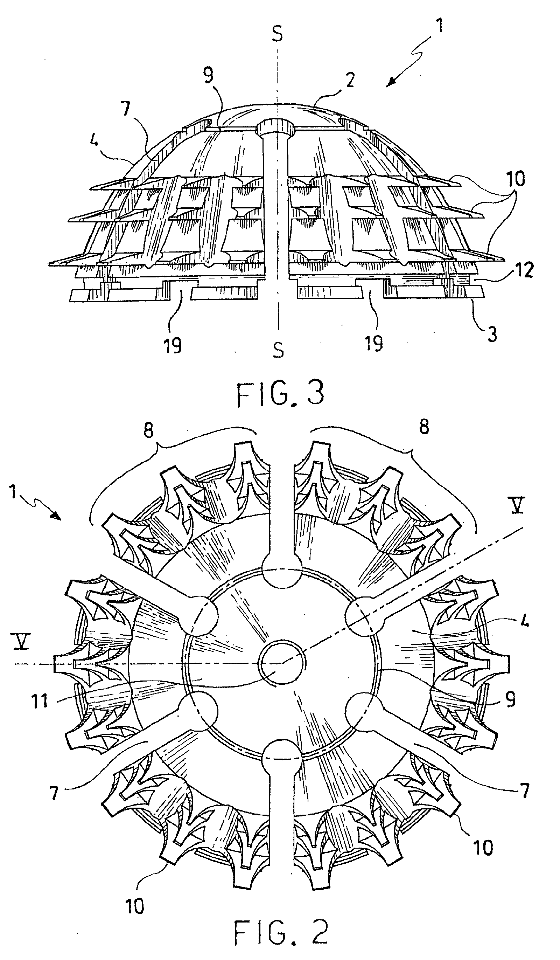

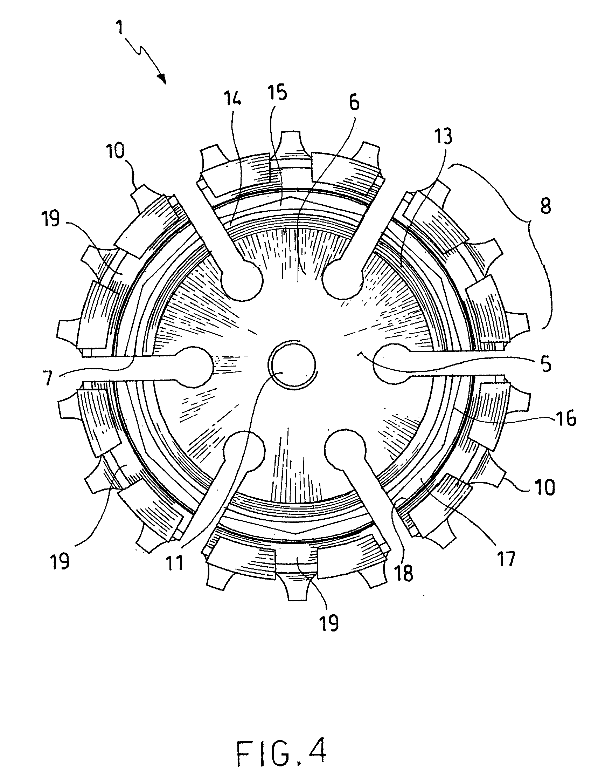

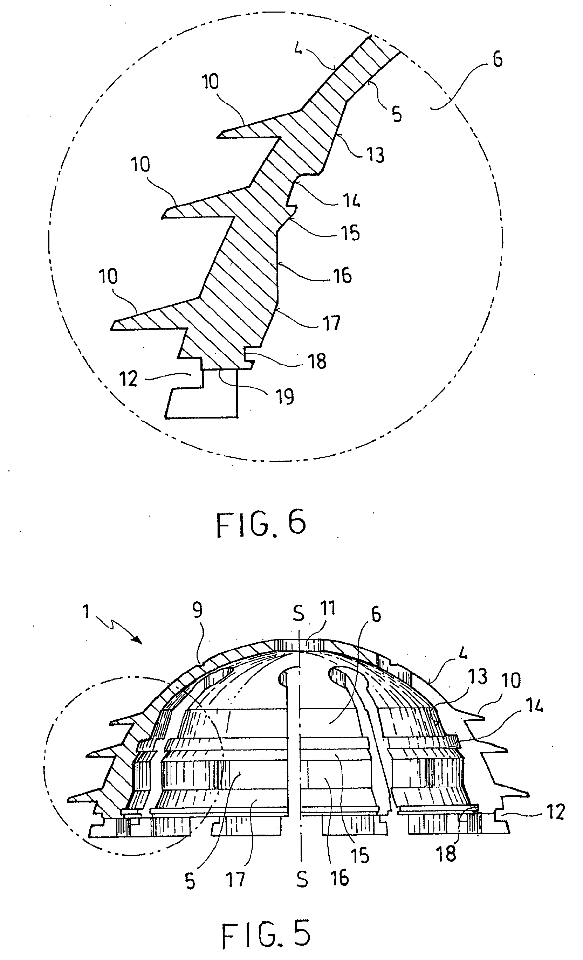

With reference to Figures 1 to 6, a socket according to the invention is indicated by 1. The socket body 1 has substantially the shape of a spherical cap, the pole 2 and annular rim 3 of which respectively form the distal and proximal end of the socket 1. The socket is substantially symmetrical with respect to an axis s, the parallel direction is defined by the section line between any plane perpendicular to the axis s and the socket body 1 and the meridian direction is defined by the section line between any plane containing the axis s and the socket 1.

The socket 1 comprises an outer surface 4 and an inner surface 5, in which the inner surface 5 bounds a proximal cavity 6. The socket 1 is provided with a plurality of meridian incisions 7 which extend from said annular rim towards the pole and all of which terminate at a common parallel, defining as many segments 8 elastically flexible in a radial direction with respect to the axis s. The ends of said meridian incisions 7 are widened and rounded. On the outer surface 4 there is further provided a substantially annular indentation 9 which extends along the parallel on which the meridian incisions 7 terminate.

From the outer surface 4, locking fins 10 project in a radial direction. Said fins 10 are disposed in three annular series parallel to the annular rim 3 and are further slightly inclined in the proximal direction of the socket 1.

At the pole 2 of the socket 1 a through hole 11 with internal thread is provided. The outer surface 4 has in proximity to the annular rim 3 an annular groove 12 inside which a clamping ring can be inserted.

The inner surface 5, in the polar region, bounds a substantially hemispherical portion of the proximal cavity 6, which is followed by a frustoconical portion 13 which widens out in the proximal direction. The proximal edge of said frustoconical portion is bounded by a ring-shaped parallel groove, hereinafter referred to as the distal groove 14. Said distal groove is followed, in the proximal direction, by a substantially annular raised portion 15 parallel to the annular rim. In the example illustrated in the drawings, the distal side of the raised portion 15 coincides with the proximal side of the distal groove 14. The entire raised portion is inclined in the distal direction of the socket.

Advantageously, the inner surface 5 between said raised portion 15 and the annular rim 3 of the socket 1 bounds a polygonal seat 16. To even greater advantage, the proximal border of said polygonal seat 16 is flared so as to form a frustoconical thrust portion 17 for widening out said segments 8.

In the embodiment described, said proximal flaring 17 occupies about half of the total meridian extent of the polygonal seat 16 and extends as far as the annular rim 3 of the socket 1.

The polygonal seat 16 itself has the shape of a regular prism, the number of sides of which is equal to twice the number of segments 8 of the socket 1.

Preferably, the edges of the polygonal seat are flared. In the preferred embodiment, said edges are rounded with a radius of 5 mm.

As can be seen from the drawings, the socket 1 has six segments 8 and said polygonal seat 16 has in cross-section a dodecagonal shape in which the corners of the dodecagon are advantageously disposed respectively at said meridian incisions 7 and at the centre of each of said segments 8.

A second parallel groove, referred to as the proximal groove 18, extends along the proximal margin of the frustoconical thrust portion 17 at the annular rim 3 of the socket.

The annular rim of the socket 1 is interrupted by radial indentations 19 which involve the entire thickness of the rim and which are provided in a position aligned with the corners of said polygonal seat 16. The depth of said radial indentations 19 is limited so as not to interfere either with the proximal flaring 17 of the polygonal seat 16 or with the proximal groove 18. The socket 1 according to the invention is produced in biocompatible and osteo-integrable material, preferably in titanium or titanium alloys.

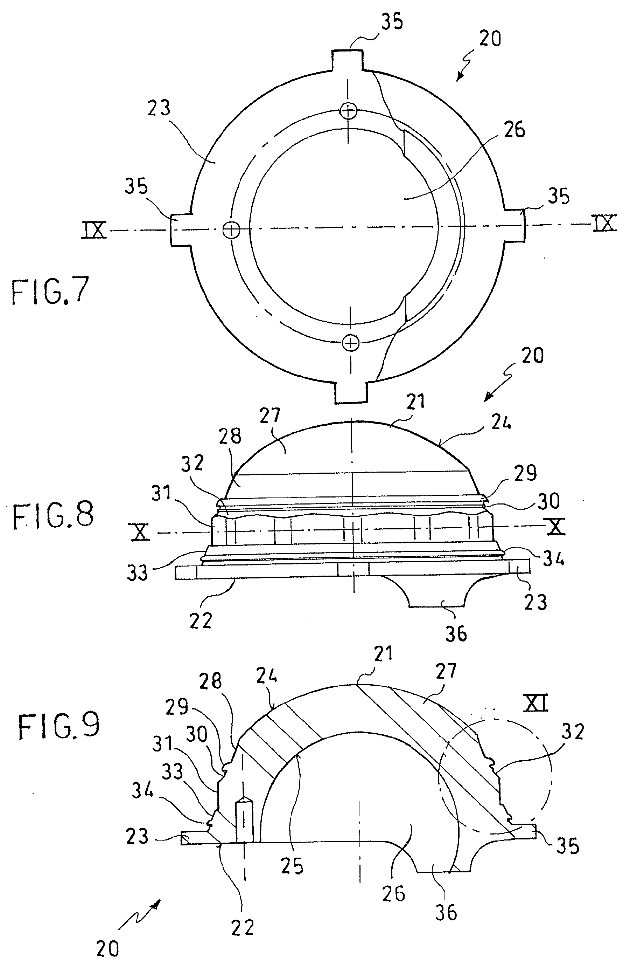

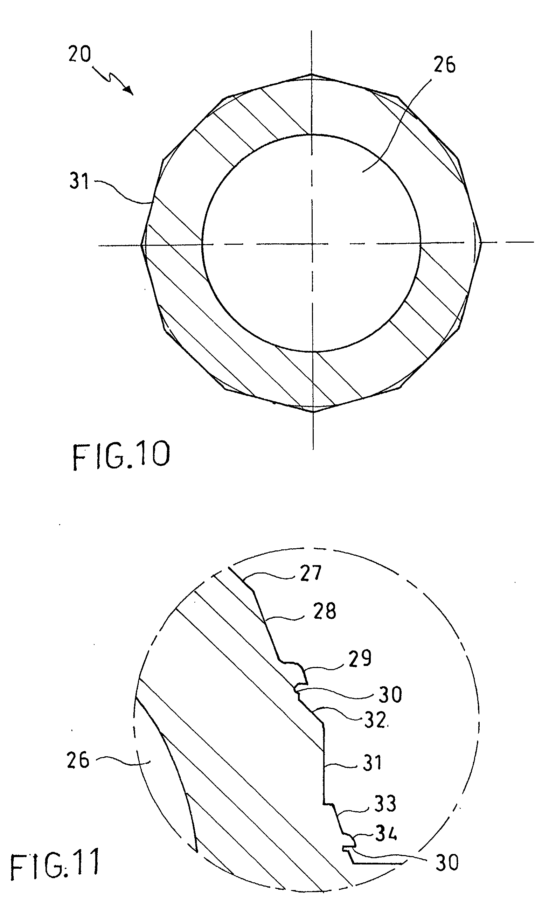

A polyethylene joint insert 20 according to the invention is illustrated in the drawings in Figures 7 to 11.

The insert 20 has substantially the shape of a spherical cap, the pole 21 and annular rim 22 of which respectively form the distal and proximal end of the joint insert 20.

The joint insert comprises a proximal flange 23, disposed at said annular rim 22, and also an outer surface 24 and an inner surface 25. The inner surface 25, produced in such a manner as to be smooth, bounds a substantially spherical pit, hereinafter referred to as the articulation seat 26.

The outer surface 24 has in the polar region a substantially hemispherical portion 27 which is followed by a frustoconical portion 28 which widens in the proximal direction of the joint insert.

The proximal edge of said frustoconical portion 28 is followed in the proximal direction by a first annular relief, parallel to said annular rim 22 and hereinafter referred to as the distal relief 29. The distal relief 29 has a substantially trapezoidal shape in cross-section, in which the distal side has a greater inclination than the proximal side. The result is that the entire distal relief 29 is inclined in the proximal direction.

Alongside said distal relief 29, in the proximal direction, an indentation 30 is provided which extends over the entire circumference of the joint insert 20. The indentation 30 has a rounded shape in cross-section.

Advantageously, the outer surface 24 has between said indentation 30 and said proximal flange 23 a polygonal portion 31, complementary and connectable to the aforesaid polygonal seat 16 provided in the socket 1.

To even greater advantage, the distal border of said polygonal portion 31 is flared so as to form a frustoconical wedge portion 32, adapted to push against said frustoconical thrust portion 17 of the socket to widen out the segments 8.

In the embodiment described here, said frustoconical wedge portion 32 defines an angle of 135° with said polygonal portion 31.

The polygonal portion 31 has the shape of a regular prism, having all the geometric characteristics described previously with reference to the aforesaid polygonal seat 16 of the socket. The element complementary to the frustoconical thrust portion 17 of the socket 1 is provided by a proximal frustoconical portion 33 which widens in the proximal direction and the distal border of which substantially coincides with the proximal border of the polygonal portion 31.

The geometric relationships between the polygonal portion 31 and the proximal frustoconical portion 33 of the insert 20 correspond substantially to those between the polygonal seat 16 and the frustoconical thrust portion 17 of the socket 1 described previously.

A second annular relief, parallel to the annular rim 22 and referred to hereinafter as the proximal relief 34, extends close to the proximal margin of the proximal frustoconical portion 33, which in its turn adjoins said proximal flange 23. The proximal relief 34 is also flanked by an annular indentation 30 parallel to the annular rim 22.

As can be seen for example from Figure 7, the proximal flange 23 of the insert 20 has four radial projections 35, distributed with a pitch having an angle of 90° between them and aligned respectively with a corner of said polygonal portion 31.

The proximal flange 23 additionally has a reinforced region 36 bulging in the proximal direction, which renders the articulation seat 26 non-dislocating.

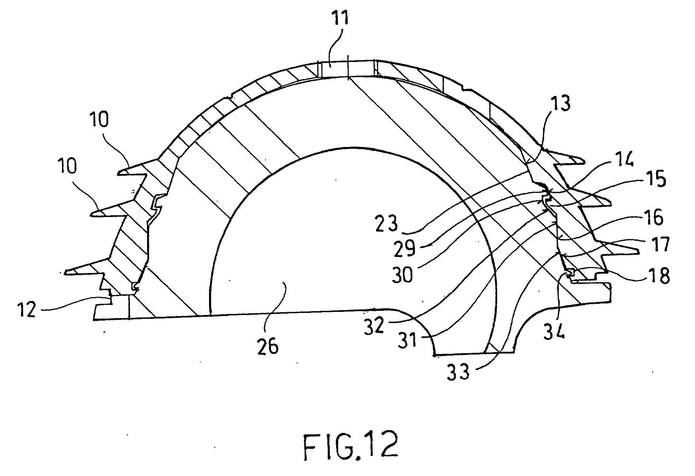

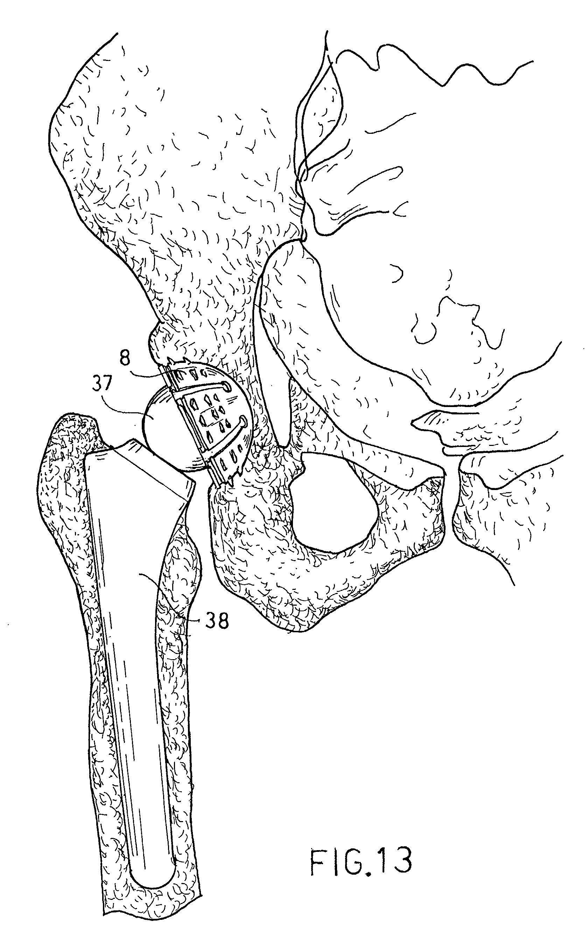

The functioning of the acetabular cup 1 for a hip joint prosthesis is described hereinafter with reference to Figures 12 and 13.

The precompressed socket 1, circled at the groove 12 by a clamping ring, not shown here, is positioned in a hemispherical seat, milled in the bone of the pelvis at the hip joint, by means of a suitable instrument in the form of a handle with a handgrip, which engages in the threaded hole 11 of the socket.

After correct positioning of the socket 1 within the natural seat, the clamping ring is cut, permitting the segments 8 to widen out and embed themselves with their locking fins 10 at least partially in the surrounding bony tissue.

At this point, widening out of the segments 8 is not yet complete. The final part of the expansion of the socket 1 remains up to the joint insert 20.

Said joint insert 20 is inserted into the proximal cavity 6 of the socket 1, aligning during insertion the radial projections 35 of the proximal flange 23 with the radial indentations 19 provided on the annular rim 3 of the socket. During fitting, the frustoconical wedge portion 32 of the insert pushes against the frustoconical thrust portion 17 of the socket, further widening out the segments 8. This widening out, and therefore flexion of the segments 8, is facilitated by the reduction in section due to the final widening out of the meridian incisions 7, and also by the annular indentation 9.

The insertion of the joint insert 20 into the socket 1 may be facilitated by means of a positioning instrument which engages with pins in suitable holes present in the proximal flange 23 of the insert 20.

From Figure 12 it is possible to observe a joint insert according to the invention positioned within a socket according to the invention. During insertion, the annular reliefs 29, 34 on the outer surface 24 of the insert flex in the proximal direction. Owing to the indentations 30, said reliefs are not likely to be sheared or plastically deformed, but they can deform freely and substantially elastically, temporarily filling the voids of the indentations 30.

As soon as the position of the reliefs 29, 34 corresponds to the position of the grooves 14, 18, the polygonal portion 31 is received by the polygonal seat 16 and said reliefs 29, 34 reassume their natural shape and snap into the grooves 14, 18, preventing the escape of the joint insert 20 from the socket 1.

Owing to the complementary nature of the polygonal seat 16 of the socket and the polygonal portion 31 of the insert 20, rotation of the insert within the socket is prevented.

The joint insert 20 itself is intended to house in its articulation seat 26 a spherical head 37 of a femoral prosthesis 38.

The radial indentations 19, present on the annular rim of the socket 1, besides acting as a positioning reference, also allow the access of implements suitable for extracting the joint insert 20 from the proximal cavity 6 of the socket 1 in the event of replacement or repositioning.

The acetabular cup 1 and also the joint insert 20 for a hip joint prosthesis have numerous advantages.

Owing to the particular conformation of the socket according to the invention, and also of the joint insert according to the invention, it is possible to remedy the drawbacks cited with reference to the prior art.

Firstly, any unwanted relative movement, either rotational or translational, between the joint insert 20 and the socket 1 is prevented. Consequently, a play of forces between the artificial implant and the natural tissue is guaranteed which favours the correct functioning and a long service life of the hip joint prosthesis.

The socket 1 according to the invention further guarantees a particularly strong anchorage of the hip joint prosthesis in the bone of the pelvis.

The provision of three annular series of fins on the outer surface prevents movement or the escape of the socket from the natural seat, in particular in the presence of an altered bony tissue, for example of the sclerotic type.

Obviously, an expert in the field, for the purpose of satisfying contingent and specific requirements, may apply to the acetabular cup and the joint insert for a hip joint prosthesis according to the invention further modifications and variants, all, however, contained within the scope of protection of the invention as defined by the following claims.

## Claims

1. A socket (1) for a hip joint prosthesis, having substantially the shape of a spherical cap, wherein the polar region (2) and the annular rim (3) of the cap respectively constitute the distal and proximal end of the socket (1), said socket (1) having an outer surface (4) and an inner surface (5) which bounds a proximal cavity (6), said socket (1) being further provided with a plurality of meridian incisions (7) which define as many segments (8) elastically flexible in a radial direction, said inner surface (5) of the socket (1) being provided with one or more grooves (14, 18) parallel to said annular rim (3), capable of receiving corresponding reliefs (29, 34) of a joint insert (20), and one or more frustoconical portions (13, 17) capable of forming bearing surfaces for corresponding frustoconical portions (28, 33) of said joint insert (20), **characterised in that** said inner surface (5) additionally comprises a portion bounding a polygonal seat (16), adapted to engage with a polygonal portion (31) of said joint insert (20) by means of a form fit.

2. A socket (1) according to claim 1, wherein said inner surface (5) comprises a frustoconical thrust portion (17) which constitutes a thrust surface for widening out said segments (8).

3. A socket (1) according to claim 2, wherein said frustoconical thrust portion (17) is a proximal flaring of said polygonal seat (16).

4. A socket (1) according to any one of the preceding claims, wherein said polygonal seat (16) forms a regular prismatic seat.

5. A socket (1) according to any one of the preceding claims, wherein the number of sides of said polygonal seat (16) is equal to twice the number of segments (8) of the socket.

6. A socket (1) according to claim 5, wherein the corners of said polygonal seat (16) are disposed at the meridian incisions (7) and at the centre of each of the segments (8).

7. A socket (1) according to any one of the preceding claims, wherein the edges of said polygonal seat (16) are rounded.

8. A socket (1) according to any one of the preceding claims, wherein said polygonal seat (16) is disposed in proximity to the annular rim (3).

9. A socket (1) according to claim 3, wherein the meridian extent of said proximal flaring of said polygonal seat (16) is greater than one third of the total meridian extent of said polygonal seat (16).

10. A socket (1) according to claim 9, wherein the meridian extent of said proximal flaring of said polygonal seat (16) is about half the total meridian extent of said polygonal seat (16).

11. A socket (1) according to claim 6, wherein said segments (8) are six in number and said polygonal seat (16) has a dodecagonal shape in cross-section.

12. A socket (1) according to any one of the preceding claims, wherein a proximal groove (18) of said grooves (14, 18) extends in the region of the frustoconical thrust portion (17).

13. A socket (1) according to claim 12, wherein said proximal groove (18) extends along the proximal margin of the frustoconical thrust portion (17).

14. A socket (1) according to claim 13, provided with a distal groove (14) of said grooves (14, 18), which extends in the region adjacent to the distal margin of said polygonal seat (16).

15. A socket (1) according to claim 14, provided with a substantially annular raised portion (15) parallel to the annular rim (3), which is disposed on the inner surface (5) between said polygonal seat (16) and said distal groove (14).

16. A socket (1) according to claim 15, wherein a frustoconical portion (13) of said frustoconical portions (13, 17) follows on adjacent to said distal groove (14) in the distal direction.

17. A socket (1) according to any one of the preceding claims, wherein the ends, facing towards the polar region (2) of the socket, of said meridian incisions (7) are widened and rounded.

18. A socket (1) according to any one of the preceding claims, wherein a substantially annular indentation (9) extends on the outer surface (4) along the parallel on which said meridian incisions (7) terminate.

19. A socket (1) according to any one of the preceding claims, comprising locking fins (10), disposed on said outer surface (4) and inclined in the proximal direction.

20. A socket (1) according to claim 19, wherein said locking fins (10) are disposed in a region which, starting from the annular rim (3), extends beyond half the height of the socket in the distal direction.

21. A socket (1) according to claim 20, wherein said locking fins (10) form three annular series which extend parallel to the annular rim (3).

22. A socket (1) according to any one of the preceding claims, comprising on said outer surface (4) at the annular rim (3) an annular groove (12), capable of receiving a clamping ring for clamping the segments (8) in the collected implant position.

23. A socket (1) according to any one of the preceding claims, comprising alignment means (19), capable of ensuring correct positioning of said joint insert (20) within said proximal cavity (6).

24. A socket (1) according to claim 23, wherein said alignment means (19) comprise radial indentations (19) on said annular rim (3).

25. A socket (1) according to claim 24, wherein said radial indentations (19) are disposed aligned with the corners of said polygonal seat (16).

26. A socket (1) according to any one of the preceding claims, produced in biocompatible and osteo-integrable material.

27. A socket (1) according to claim 26, produced in titanium or alloys thereof.

28. A joint insert (20), having substantially the shape of a spherical cap with a distal polar region (21) and a proximal annular rim (22) and comprising an articulation seat (26) capable of receiving a spherical head (37) of a femoral prosthesis (38), an outer surface (24) being provided with one or more annular reliefs (29, 34) parallel to said annular rim (22) and capable of engaging in corresponding grooves (14, 18) provided in a socket (1) for the connection of the joint insert (20) to the socket (1), and one or more frustoconical portions (28, 33) capable of forming bearing surfaces for corresponding frustoconical portions (13, 17) of said socket (1), **characterised in that** from said outer surface (24) there extends a polygonal portion (31) complementary to a polygonal seat (16) provided in said socket (1).

29. A joint insert (20) according to claim 28, wherein said outer surface (24) comprises a frustoconical wedge portion (32), adapted to push against a thrust surface (17) of the socket to widen out the socket (1).

30. A joint insert (20) according to claim 29, wherein said frustoconical wedge portion (32) is formed by a distal flaring of said polygonal portion (31).

31. A joint insert (20) according to any one of claims 28 to 30, wherein said polygonal portion (31) forms a regular prismatic portion.

32. A joint insert (20) according to any one of claims 28 to 31, wherein said polygonal portion (31) has a dodecagonal shape in cross-section.

33. A joint insert (20) according to any one of claims 28 to 32, wherein the edges of said polygonal portion (31) are rounded.

34. A joint insert (20) according to one of claims 28 to 33, wherein said one or more reliefs (29, 34) are inclined in the proximal direction.

35. A joint insert (20) according to one of claims 28 to 34, comprising a proximal flange (23) which extends along said proximal annular rim (22).

36. A joint insert (20) according to claim 35, wherein said proximal flange has alignment means (35) for aligning the polygonal portion (31) of the insert with the complementary polygonal seat (16) of the socket (1).

37. A joint insert (20) according to claim 36, wherein said alignment means (35) comprise one or more radial projections (35).

38. A joint insert (20) according to claim 37, wherein said one or more radial projections (35) are disposed aligned with the corners of said polygonal portion (31).

39. A joint insert (20) according to claim 38, wherein said radial projections (35) are four in number and are distributed at a pitch with an angle of 90° between them.

40. A joint insert (20) according to any one of claims 35 to 39, wherein said proximal flange (23) has a reinforced region (36) bulging in the proximal direction, adapted to render the articulation seat (26) non-dislocating.

41. A joint insert (20) according to any one of claims 28 to 40, produced in polyethylene.
